# FASCICULE DE BREVET EUROPEEN

(11) **EP 3 013 144 B1**
(45) Date de publication et mention de la délivrance du brevet: **02.03.2022**
(21) Numéro de dépôt: 14750007.8
(22) Date de dépôt: 27.06.2014
(51) Int. Cl.: A01N 1/02

(54) **UTILISATION D'UN FILTRE POUR DECONGELER DES CELLULES**
VERWENDUNG EINES FILTERS ZUM AUFTAUEN VON ZELLEN
USE OF A FILTER FOR THAWING CELLS

(30) Priorité: 27.06.2013 WO PCT/IB2013/055286
(43) Date de publication de la demande: 04.05.2016
(73) Titulaire: IBSA PHARMA SAS, 06600 Antibes (FR)
(72) Inventeur: VACHER, Dominique, F-06250 Mougins (FR); GOUZE, Jean-Noël, F-06220 Vallauris (FR); GUILLEMIN, Yannis, 06510 Gattierres (FR)
(74) Mandataire: Santarelli
(86) Numéro de dépôt international: PCT/IB2014/062664
(87) Numéro de publication internationale: WO 2014/207716

(56) Documents cités:
- WO-A1-96/30274
- WO-A2-2005/069766
- US-A- 5 776 769
- CASTINO F ET AL: "Washing frozen red blood cell concentrates using hollow fibres", JOURNAL OF MEMBRANE SCIENCE, ELSEVIER SCIENTIFIC PUBL.COMPANY. AMSTERDAM, NL, vol. 110, no. 2, 21 février 1996 (1996-02-21), pages 169-180, XP004041725, ISSN: 0376-7388, DOI: 10.1016/0376-7388(95)00244-8
- XIAOMING ZHOU ET AL: "A Dilution-Filtration System for Removing Cryoprotective Agents", JOURNAL OF BIOMECHANICAL ENGINEERING, NEW YORK, NY, US, vol. 133, no. 2, 1 février 2011 (2011-02-01), pages 21007/1-7, XP009176440, ISSN: 0148-0731, DOI: 10.1115/1.4003317 [extrait le 2011-01-31]
- CESARE G. PEROTTI ET AL: "A new automated cell washer device for thawed cord blood units", TRANSFUSION, vol. 44, no. 6, 1 juin 2004 (2004-06-01), pages 900-906, XP055104893, ISSN: 0041-1132, DOI: 10.1111/j.1537-2995.2004.03389.x
- CALMELS B ET AL: "Preclinical evaluation of an automated closed fluid management device: CytomateTM, for washing out DMSO from hematopoietic stem cell grafts after thawing", BONE MARROW TRANSPLANTATION, NATURE PUBLISHING GROUP, GB, vol. 31, no. 9, 1 mai 2003 (2003-05-01), pages 823-828, XP002520080, ISSN: 0268-3369, DOI: 10.1038/SJ.BMT.1703905
- ARNAUD F ET AL: "Use of hollow fiber membrane filtration for the removal of DMSO from platelet concentrates", PLATELETS, TAYLOR AND FRANCIS GROUP, EDINBURGH, vol. 14, no. 3, 1 janvier 2003 (2003-01-01), pages 131-137, XP009176438, ISSN: 0953-7104, DOI: 10.1080/0953710031000092811
- ALGHAMDI ABDORRAHMAN S ET AL: "Effect of seminal plasma concentration and various extenders on postthaw motility and glass wool-Sephadex filtration of cryopreserved stallion semen", AMERICAN JOURNAL OF VETERINARY RESEARCH, AMERICAN VETERINARY MEDICINE ASSOCIATION, US, vol. 63, no. 6, 1 juin 2002 (2002-06-01), pages 880-885, XP009176435, ISSN: 0002-9645
- BENSON E E: "CRYOPRESERVATION", PRACTICAL APPROACH SERIES. PLANT CELL CULTURE, XX, XX, 1 janvier 1994 (1994-01-01), pages 147-167, XP000646465,

## Description

La présente invention concerne le domaine de la culture cellulaire. Plus précisément, elle a pour objet une méthode de décongélation de cellules plus rapide et facile que les méthodes classiques.

Dans de nombreuses situations, la maintenance des cultures de cellules nécessite leur congélation et leur conservation à une température inférieure ou égale à -130°C.

Lors de la congélation, il y a d'abord un ralentissement du métabolisme cellulaire entre la température initiale et 0°C, puis, entre 0° et -20°C, formation de cristaux de glace dans l'environnement extracellulaire, ce qui augmente la concentration en solutés du milieu de culture. De ce fait, l'eau commence à sortir des cellules pour aller dans le milieu extracellulaire partiellement congelé, ce qui initialise le processus de déshydratation et de rétrécissement cellulaires. Si le processus de refroidissement est rapide, des cristaux de glace intracellulaires se forment avant la fin du processus de déshydratation cellulaire. Ces cristaux de glace déchirent les membranes et les organelles cellulaires, entraînant la mort de la cellule pendant le processus de décongélation. Si le processus de réfrigération est lent, l'eau libre intracellulaire est expulsée de la cellule par la force osmotique, entraînant une déshydratation et un rétrécissement complets de la cellule. Ceci peut également entraîner une mort cellulaire. Cependant, lorsque la vitesse de refroidissement est suffisamment lente pour empêcher la formation de glace intracellulaire, mais suffisamment rapide pour éviter les effets de déshydratation graves, les cellules peuvent survivre aux processus de congélation et de décongélation. La zone ou fenêtre de survie est observable chez de nombreuses bactéries ou autres procaryotes, mais pour la plupart des cellules eucaryotes, elle est inexistante ou très difficile à trouver sans utiliser d'agents cryoprotecteurs. Ces agents ont peu d'effet sur les dommages causés par une congélation trop rapide (formation de cristaux de glace intracellulaires), mais préviennent ou diminuent généralement les dommages dus à la congélation lente (déshydratation et rétrécissement).

Une grande variété de produits chimiques sont utilisés assurer une cryoprotection adéquate, y compris l'acétamide de méthyle, le méthanol, l'éthylène glycol et la polyvinylpyrrolidone. Cependant, le diméthylsulfoxyde (DMSO) et le glycérol sont les plus pratiques et les plus largement utilisés. Plusieurs de ces agents, bien qu'assurant une excellente cryoprotection, ont des effets secondaires toxiques sur les cultures, rendant leur utilisation difficile. Le DMSO est le plus souvent utilisé à une concentration finale de 5 à 15% (v/v). Certaines lignées cellulaires sont affectées par un contact plus ou moins prolongé avec le DMSO. Cet effet peut être réduit en ajoutant le DMSO à la suspension cellulaire à 4°C lors de la congélation, et en le retirant immédiatement lors de la décongélation. Le glycérol est généralement utilisé à une concentration finale entre 5 et 20% (v/v). Bien qu'étant moins toxique pour les cellules que le DMSO, le glycérol cause fréquemment des problèmes osmotiques, surtout après décongélation.

Aussi, quel que soit l'agent cryoprotecteur utilisé, il est nécessaire de le diluer ou de l'éliminer rapidement lors de la décongélation des cellules. Dans certains cas, il est indispensable d'éliminer le plus complètement possible l'agent cryoprotecteur. Ceci est le cas, par exemple, lorsque les cellules décongelées sont très fragiles ou destinées à être mises en coculture avec des cellules fragiles, comme dans le cas des co-cultures avec des embryons. Ceci est également le cas dans les applications de thérapie cellulaire, lorsque les cellules décongelées sont destinées à être injectées dans un patient ; en effet, des effets secondaires ont été observés après injection de cellules hématopoïétiques dans une solution contenant du DMSO (hypotension, trouble du rythme cardiaque, convulsions..). Cette élimination est actuellement réalisée par lavage(s). Le plus souvent, après une décongélation rapide en plaçant le contenant dans un bain-marie à 37°C, les cellules sont transférées, de façon stérile, dans un tube de centrifugation, pour effectuer une ou plusieurs étapes de centrifugation avec élimination du surnageant et remise en suspension dans un milieu de culture ne contenant pas d'agent cryoprotecteur.

La multiplication des étapes de pipetage, centrifugation, élimination du surnageant et remise en suspension entraîne un risque de contamination des cellules décongelées, et une immobilisation relativement longue d'un technicien ainsi que de matériels tels que centrifugeuse, hotte de culture, etc. Un objectif de la présente invention est de proposer un procédé de décongélation cellulaire simplifié, permettant d'éliminer les agents cyoprotecteurs très rapidement et en limitant le nombre de manipulations. Ceci est obtenu en utilisant un dispositif filtrant tel que les cellules soient retenues sur un filtre traversé par le milieu de congélation. L'invention est exposée dans le jeu de revendications ci-joint.

La présente porte donc sur un procédé de décongélation cellulaire, comprenant une étape de décongélation d'une suspension cellulaire constituée d'un milieu de congélation et de cellules, suivie d'une étape d'élimination dudit milieu de congélation par filtration. Dans le présent texte, une "suspension cellulaire" désigne une solution de milieu ou de tampon dans laquelle les cellules, qui peuvent être des cellules adhérentes, sont présentes de façon essentiellement individualisée, par opposition à un fragment de tissu, dans lequel les cellules sont présentes sous forme d'un amas organisé. L'étape d'élimination du milieu de congélation est effectuée sans dilution préalable de la suspension cellulaire, comme cela est fréquemment le cas, par exemple dans les procédés décrits dans les articles de Castino et Wickramashinge (Journal of Membrane Science, 1996), Xiaomin Zhou et al. (Journal of Biochemical Engineering, 2011), Perrotti et al. (Transfusion, 2004), Calmels et al. (Bone Marrow Transplantation, 2003) et Alghamdi et al. (AJVR, 2002). Dans un procédé selon l'invention, les agents cryoprotecteurs sont éliminés en même temps que le reste du milieu de congélation, en une étape unique et rapide à l'issue de laquelle les cellules se trouvent transitoirement séparées de tout milieu, contrairement à d'autres procédés décrits, dans lesquels les agents cryoprotecteurs sont progressivement dilués par diffusion à travers une membrane comme décrit dans le brevet US 5,776,769 ainsi que dans l'article de Arnaud et al. (Platelets, 2003). Ainsi, selon l'invention, l'étape d'élimination du milieu de congélation est effectuée sans apport préalable ou simultané de milieu ou de tampon.

Selon un mode de réalisation particulier qui ne fait pas partie de la présente invention, la présente porte sur un procédé de décongélation cellulaire comprenant les étapes suivantes :
(i) placer un premier récipient, contenant une suspension cellulaire congelée, constituée d'un milieu de congélation et de cellules, à une température permettant la décongélation de la suspension cellulaire ;
(ii) transférer la suspension cellulaire du premier récipient dans un dispositif muni d'un filtre dont les pores ont un diamètre moyen compris entre 1 et 15 microns ;
(iii) placer ledit dispositif dans ou au-dessus d'un second récipient, et appliquer à la suspension cellulaire une force qui entraîne le passage du milieu de congélation à travers le filtre ;
(iv) remettre les cellules retenues sur le filtre en suspension dans une solution.

Le "premier récipient" du procédé ci-dessus, ou "le récipient" des procédés décrits plus bas dans lesquels un seul récipient est nécessaire, peut être n'importe quel récipient hermétique, stérilisable et supportant de très basses températures, tel qu'un cryotube, une poche à congélation, *etc.* Bien entendu, ce "premier récipient" peut être constitué d'un ensemble de récipients, par exemple un ensemble de 2 à 5 tubes de congélation dont le contenu sera regroupé dans le dispositif de filtration. Il est également envisageable, comme cela est décrit dans la partie expérimentale ci-dessous, de diviser le contenu d'un tube de congélation sur plusieurs filtres à centrifuger, afin d'éviter de colmater le filtre. Le milieu de congélation peut être n'importe quel milieu classiquement utilisé pour conserver les cellules, tel qu'un mélange de milieu de culture (par exemple, DMEM), de sérum (20%) ou d'un agent de substitution du sérum (tel que le KSR de Gibco) et d'un agent cryoprotecteur tel que le DMSO.

La présente porte sur un procédé de décongélation cellulaire comprenant les étapes suivantes :
(i) placer un récipient, contenant une suspension cellulaire congelée dans un dispositif muni d'un filtre, à une température permettant la décongélation de la suspension cellulaire;
(ii) appliquer à la suspension cellulaire une force qui entraîne le passage du milieu de congélation à travers le filtre;
(iii) remettre les cellules en suspension dans une solution.

Pour la mise en œuvre du procédé ci-dessus, il est nécessaire que le filtre ait été choisi ou traité pour que, lors de l'introduction de la suspension cellulaire à congeler dans le dispositif muni d'un filtre, le milieu de congélation ne traverse pas le filtre. Un exemple de traitement de filtre est décrit à l'exemple 5 ci-dessous. Il consiste à ajouter sur le filtre, avant la suspension cellulaire à congeler, une goutte d'un liquide hydrophobe. Ce liquide constitue une barrière transitoire entre le filtre et la suspension cellulaire. Lors de la décongélation, il est éliminé lors de l'application de la force d'entraînement, qui lui fait traverser le filtre. N'importe quelle solution hydrophobe compatible avec la survie des cellules peut être utilisée pour constituer cette "barrière transitoire" isolant la suspension cellulaire du filtre. A titre d'exemples, on peut citer le glycérol et toute huile stérile comme l'huile stérile dépourvue d'endotoxines utilisée pour recouvrir les embryons afin d'éviter l'évaporation du milieu de culture. Alternativement ou de façon complémentaire, un filtre constitué d'un matériau hydrophobe peut être utilisé, ainsi que tout autre filtre tel que le milieu de congélation ne passe pas au travers avant l'application d'une force d'entraînement qui est nécessairement supérieure à la force gravitationnelle g.

Classiquement, l'étape (i) des procédés ci-dessus sera réalisée en plaçant le(s) tube(s) de congélation dans un bain-marie à 37°C ou tout autre dispositif équivalent thermostaté (tel qu'un bain-marie sans eau à billes, un bloc chauffant, *etc.*) pendant une durée de quelques secondes à quelques minutes. Le transfert effectué à l'étape (ii) du premier mode de réalisation particulier décrit ci-dessus sera de préférence réalisé à l'aide d'une pipette, dans des conditions stériles, de même que la remise en suspension des cellules retenues sur le filtre, à la dernière étape des procédés décrits plus haut. Bien entendu, ces étapes peuvent être réalisées manuellement par un technicien, mais elles peuvent également être automatisées, en partie ou totalement.

Selon une mise en œuvre particulière de l'invention, le dispositif muni d'un filtre est un filtre à centrifuger.

Selon une autre mise en œuvre particulière de l'invention, le diamètre moyen des pores du filtre est compris entre 2 et 10 microns.

Selon une autre mise en œuvre particulière de l'invention, le passage du milieu de congélation à travers le filtre est obtenu en centrifugeant le dispositif muni d'un filtre, placé dans le récipient. Par exemple, cette étape peut être réalisée en centrifugeant le dispositif muni d'un filtre pendant une durée inférieure ou égale à 10 minutes (par exemple un simple "pulse", 10 secondes, 30 secondes, 1 minute, 3 minutes, 5 minutes ou davantage), à une vitesse permettant d'appliquer à la suspension cellulaire une accélération comprise entre 50 et 500 g, de préférence entre 100 et 200 g. Alternativement, cette étape peut être réalisée en appliquant une surpression du côté du filtre contenant la suspension cellulaire, et/ou une dépression de l'autre côté du filtre.

Selon une mise en œuvre particulière du procédé selon l'invention, la quantité de cellules transférées à l'étape (ii) ou présente dès l'étape (i) dans chaque dispositif de filtration est comprise entre10³ et 5×10⁸ cellules, de préférence entre 10⁴ et 10⁷ cellules par dispositif de filtration.

Selon une mise en œuvre particulière du procédé selon l'invention, l'étape de remise en suspension des cellules est réalisée en effectuant des pipetages à l'aide d'une pipette P200 ou P1000. Plusieurs cycles d'aspiration / refoulement peuvent être pratiqués, de façon dissocier les amas cellulaires éventuellement formés.

La présente invention porte également sur une trousse de décongélation cellulaire, comprenant un récipient contenant une suspension cellulaire congelée dans un dispositif muni d'un filtre dont les pores ont un diamètre moyen compris entre 1 et 15 microns, de préférence entre 2 et 10 microns, ledit dispositif étant stérile.

A titre d'exemples non limitatifs de cellules qui peuvent être inclues dans une telle trousse, on peut citer les cellules suivantes : VERO, L929, 3T3, Ishikawa, HeLa229, Jurkat, K562, COS-7... La quantité de cellules présentes dans la trousse dépendra bien sûr de l'application visée. La trousse peut, par exemple, contenir 1, 2, 3, 4, 5 tubes ou davantage, contenant chacun 2×10⁶ cellules VERO.

Selon un mode de réalisation particulier des trousses de l'invention, le dispositif muni d'un filtre est un filtre à centrifuger. En particulier, la trousse peut contenir 1, 2, 3, 4 filtres à centrifuger stériles, emballées individuellement ou par groupe de 2 ou davantage. Ces filtres peuvent avoir été stérilisés par tout moyen approprié, par exemple par irradiation aux rayons gamma.

Les trousses selon la présente invention sont particulièrement avantageuses pour mettre en œuvre des protocoles nécessitant l'utilisation de cellules nourricières, comme cela est le cas pour la culture d'embryons, notamment dans le cadre des fécondations *in vitro* (FIV). Une trousse prête à l'emploi pour la décongélation et la préparation de cellules nourricières telles que les cellules VERO a en effet pour but de faciliter la tâche des praticiens dans leurs actes pour favoriser le développement d'embryons jusqu'au stade blastocyste et l'implantation de ceux-ci, dans le domaine humain (thérapeutique), mais également pour des applications vétérinaires, dans un but de sélection et /ou reproduction, plus particulièrement chez les bovins, les ovins et les équins.

Selon un mode de réalisation particulier des trousses de l'invention, la trousse est destinée à favoriser la culture d'embryons ; outre les cellules congelées et le(s) dispositif(s) de filtration, une telle trousse peut également comprendre un ou plusieurs des éléments suivants : des pointes stériles de 200µl et/ou 1000µl, un support de culture pour la fécondation *in vitro,* des tubes stériles et des pipettes stériles. A titre d'exemple de support de culture adapté à la FIV, on peut citer les boites de Petri et la plaque 4 puits BD Falcon^{™} ou NUNC, pour la FIV, qui sont fabriquées à partir de polystyrène vierge cristallin testé selon les standards USP Class VI. Dans le cadre de la présente invention, une trousse destinée à favoriser la culture d'embryons peut également comprendre un milieu de rinçage et/ou un milieu de culture embryonnaire et/ou un milieu de culture pour blastocystes et/ou un milieu de culture prolongée (par exemple CCM^{™}).

Les trousses selon la présente invention sont également avantageusement utilisées pour mettre en œuvre des protocoles de maturation *in vitro* des ovocytes (MIV), comme cela est pratiqué pour surmonter une infertilité due à un syndrome des ovaires polykystiques (OPK), ou avant une chimiothérapie.

Selon un mode de réalisation particulier des trousses de l'invention, la trousse est destinée à favoriser la maturation *in vitro* des ovocytes ; outre les cellules congelées et le(s) dispositif(s) de filtration, une telle trousse peut également comprendre un ou plusieurs des éléments suivants : des pointes stériles de 200µl et/ou 1000/µl, un support de culture pour la maturation *in vitro* des ovocytes, des tubes stériles et des pipettes stériles. A titre d'exemple de support de culture adapté à la MIV, on peut citer les boites de Petri et la plaque 4 puits BD Falcon^{™} ou NUNC, pour la FIV, qui sont fabriquées à partir de polystyrène vierge cristallin testé selon les standards USP Class VI. Dans le cadre de la présente invention, une trousse destinée à favoriser la maturation *in vitro* des ovocytes peut également comprendre un milieu de rinçage et/ou un milieu de culture pour maturation ovocytaire (par exemple enrichi en FSH et/ou oestradiol et/ou gonadotrophines).

Un autre aspect qui ne fait pas partie de la présente invention porte sur une trousse de congélation cellulaire, qui comprend :
(i) un dispositif muni d'un filtre ; et
(ii) un récipient disposant de moyens de fermeture hermétique, capable de contenir le dispositif muni d'un filtre (i),
telle que le filtre est choisi de façon à ce que le milieu de congélation ne le traverse pas en l'absence d'une force d'entraînement supérieure à la force gravitationnelle (par exemple, grâce à des propriétés hydrophobes), et/ou telle que la trousse comprend une solution hydrophobe (non miscible dans l'eau) capable de traverser le filtre uniquement lorsqu'une force d'entrainement supérieure à la force gravitationnelle lui est appliquée. Cette solution hydrophobe, qui peut être, par exemple, une solution de glycérol ou une huile stérile dépourvue d'endotoxines, est destinée à constituer une barrière transitoire entre le filtre et la solution cellulaire à congeler, avant l'introduction de la suspension cellulaire à congeler dans le dispositif muni d'un filtre.

Dans les trousses de congélation cellulaire ne faisant pas partie de l'invention, les moyens de fermeture hermétique du récipient destiné à recevoir le dispositif muni d'un filtre comprennent, de préférence, un bouchon à vis. Selon une mise en œuvre préférée, le récipient est semblable aux tubes de congélation cellulaire, adapté pour contenir filtre tel que ceux figurant dans les unités de filtration par centrifugation Ultrafree ^{®} (Millipore ^{®}), l'ensemble "récipient hermétique + filtre" constituant, le cas échéant, une unité de filtration par centrifugation. Selon les formats et utilisations, les trousses peuvent contenir une ou plusieurs (2, 5, 10, 20 ou davantage) unités de filtrations, stériles ou stérilisables.

Les exemples suivants illustrent l'invention sans toutefois limiter son étendue.

### Légendes des figures

**Figure 1**: Nombre de cellules récupérées après décongélation et centrifugation dans les tubes Ultrafree.
**Figure 2**: Rendement et viabilité cellulaire des cellules décongelées et centrifugées dans les tubes Ultrafree.
**Figure 3** : Nombre de cellules par puits au cours de la culture de cellules (cellules décongelées selon la technique des tubes Ultrafree et ensemencées en plaque 4 puits).
**Figure 4** : Viabilité cellulaire au cours de la culture (cellules décongelées selon la technique des tubes Ultrafree et ensemencées en plaque 4 puits).
**Figure 5** : viabilité de cellules VERO après 10 jours de congélation à -80°C dans des tubes Ultrafree^{®}-CL dont les membranes ont été recouvertes de 50 µL de glycerol.
**Figure 6** : Amplification cellulaire après congélation dans les tubes Ultrafree^{®}-CL.

### Exemples

### Exemple 1 : Utilisation des colonnes de décongélation (= filtres à centrifuger) pour la décongélation et mise en culture de cellules cryoconservées

### Description des filtres à centrifuger

- filtres à centrifuger Ultrafree-CL SV de Millipore :
- référence : UFC40SV25 filtres à centrifuger Ultrafree-CL SV 5.0
- filtre pour microfiltration
- filtre en PVDF (polyfluorure de vinylidène) hydrophile
- taille des pores du filtre : 5.0µm
- dispositif en polypropylène
- non stérile

### Processus préalable sur les filtres à centrifuger

Pour les rendre utilisables en culture cellulaire, ces filtres à centrifuger ont subi le traitement suivant :
- emballage en polyéthylène individuel soudé, (emballage peut être par plusieurs unités éventuellement)
- stérilisation par irradiation aux rayons gamma à 25kGy (une quantité de 8 kGy est suffisante).

L'embryotoxicité de ce dispositif ainsi traité a été testée par des tests dits « *Mouse embryo assay* », démontrant l'innocuité des filtres à centrifuger sur le développement de l'embryon de souris jusqu'au stade blastocysyte.

Cellules : lignée cellulaire de rein de singe : cellules Véro, cryoconservées en vapeur d'azote, à 2×10⁶ cell/ampoule, avec 1ml de milieu de congélation : milieu de culture MEMα (80%) / Sérum de Veau Fœtal (10%) / Dimethyl sulfoxide (10%).

### Protocole :

Décongélation des cellules cryoconservées
➢ A partir d'une ampoule de cellules VERO cryoconservées (Vero WHO, catalogue n°88020401 à l'ECACC)
➢ Placer l'ampoule 1 minute au bain marie à 37°C
➢ Homogénéiser le contenu de l'ampoule
➢ Déposer 500 µL de suspension cellulaire dans chacun des deux tubes UltraFree stérilisés
➢ Ajouter 1 mL de milieu MEMa dans chacun des filtres à centrifuger
➢ Centrifuger les filtres à centrifuger Ultrafree à 5 min à 100 g
➢ Pendant la centrifugation :
   ∘ Préparer 1 tube 15 mL (tube T1)
   ∘ Préparer 1 tube à hémolyse contenant 100µL de bleu trypan (tube T2)

Récupération des cellules dans les tubes Ultrafree à la fin de la centrifugation :
➢ Déposer 500µL de Milieu CCM^{™}-30 (Vitrolife, Göteborg, Suède) sur le filtre du premier tube Ultrafree
➢ Réaliser une série de 5 cycles aspiration/refoulement sur la totalité du filtre
➢ Déposer les 500µl de suspension obtenue dans le tube T1
➢ Déposer à nouveau 500µL de Milieu CCM sur le filtre du même premier tube Ultrafree
➢ Réaliser une série de 5 cycles aspiration/refoulement sur la totalité du filtre
➢ Déposer les 500µl de suspension obtenue dans T1

Réaliser exactement la même manipulation sur le deuxième filtre à centrifuger.
➢ Homogénéiser la suspension de T1: « Suspension SI » (volume 2ml)
➢ Prélever et déposer 100 µL de la suspension cellulaire SI dans T2 pour comptage
➢ Réaliser le comptage cellulaire, et viabilité, en déposant de la suspension cellulaire entre lame et lamelle d'un hématimètre
➢ Ajuster la concentration cellulaire à de 480 000 cellules/ml avec du milieu CCM
➢ Ensemencer 4 puits d'une plaque FIV à 240 000 cellules/puits (soit 500µL/puits).

Suivi de la culture à J1
➢ Observer les cultures
➢ Trypsiner un puits et effectuer un comptage/viabilité (+ Bleu trypan)
   ∘ Rincer les puits avec 1 ml de PBS
   ∘ Aspirer le PBS
   ∘ Ajouter 300µl de trypsine et placer les cultures 5 minutes à 37°C
   ∘ Aspirer et refouler (l'aide d'une P200 et de pointes de 200 µL) la suspension jusqu'à dissolution complète des amas cellulaires
   ∘ Inhiber l'action de la trypsine en ajoutant 200 µL de milieu pour cellules VERO
   ∘ Aspirer et refouler la suspension jusqu'à dissolution complète des amas cellulaires
   ∘ Compter les cellules VERO à l'aide d'un hématimètre.
➢ Rincer les autres puits avec 1mL de PBS/puits.
➢ Ajouter 1mL de CCM/puits
➢ Incuber les plaques à 37°C+5%CO₂

- Suivi de la culture à J2 = observation des cultures et trypsination d'un puits selon la description idem J1 (pas de changement de milieu)
- Suivi de la culture à J5 = observation des cultures ettrypsination d'un puits selon la description idem J1 (pas de changement de milieu)
- Suivi de la culture à J7 = observation des cultures et trypsination d'un puits selon la description idem J1 (pas de changement de milieu)

### Résultats

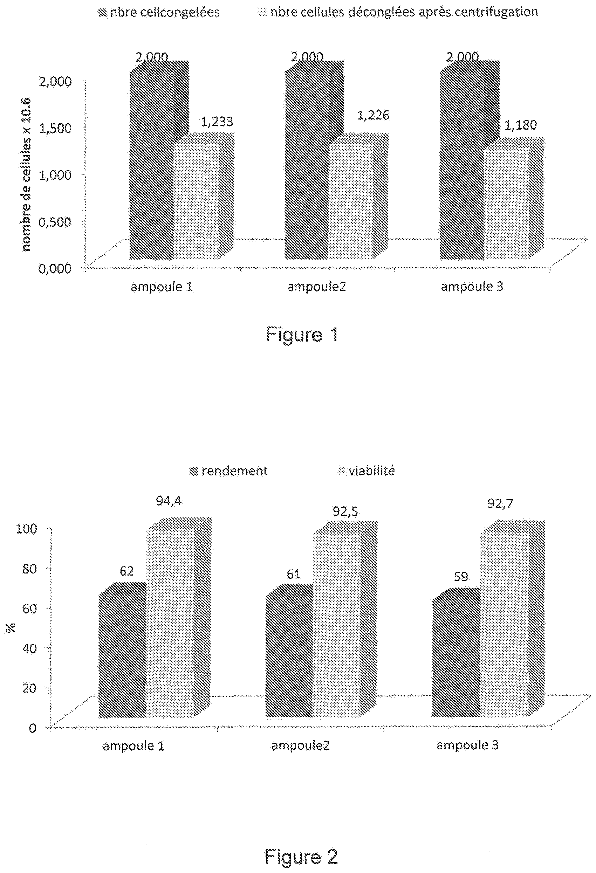

L'expérience a été réalisée sur trois ampoules de cellules VERO, les résultats obtenus sont résumés dans les graphes des Figures 1 et 2.

Les résultats obtenus montrent que le système de décongélation des cellules VERO par les filtres à centrifuger permet de récupérer plus d'un million de cellules vivantes par ampoule de cellules décongelées. La viabilité cellulaire est très satisfaisante (supérieur à 90%), et le rendement est satisfaisant également (supérieur à 50%).

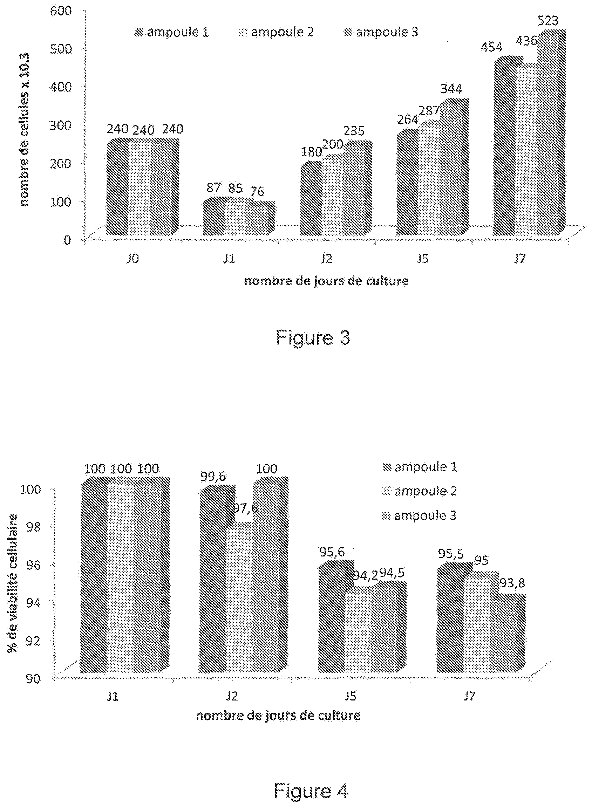

Les résultats de remise en culture des cellules VERO décongelées selon cette technique des filtres à centrifuger Ultrafree montrent une très bonne viabilité cellulaire au cours du temps supérieur à 90% sur 7 jours de culture (Figures 3 et 4).

Par ailleurs, le nombre de cellules par puits au cours du temps montrent une bonne croissance cellulaire à partir du jour 1. La chute du nombre de cellules entre J0 et J1 après décongélation et remise en culture, est observée dans beaucoup de types cellulaires ; elle s'explique par le fait que certaines cellules puissent être viables à la décongélation sans être capables de proliférer par la suite.

A partir du jour J1, le nombre de cellules par puits augmente progressivement, pour finalement atteindre près de 0.5 millions par puits au jour 7 de culture, soit le double du nombre de cellules ensemencées.

Les cellules Véro décongelées selon la technique des tubes Ultrafree sont donc viables et capables de proliférer en culture pendant 7 jours. Les résultats obtenus sur trois essais montrent que cette technique est fiable et reproductible.

### Exemple 2 : utilisation des colonnes de décongélation (filtres à centrifuger) dans un Kit prêt à l'emploi pour la décongélation et la préparation de cellules VERO pour le développement d'embryon de souris jusqu'au stade blastocyste

### Protocole :

- Au jour J0 : décongeler une ampoule de cellules VERO et ensemencer 4 puits d'une plaque FIV, selon le protocole décrit dans l'exemple n°1.
- Au jour J1 : rincer chaque puits avec 1 ml d'une solution tampon, et ajouter 1 ml de milieu CCM.
- déposer des embryons de souris au stade zygote : 10 embryons / puits.
- Au jour J2, évaluer le % de zygotes ayant atteint le stade embryons deux cellules.
- Au jour 5 : évaluer le % d'embryons 2 cellules ayant atteint le stade blastocyste.
- Au jour 6 : évaluer le % d'embryons 2 cellules ayant atteint le stade blastocyste.

### Résultats

L'expérience a été réalisée sur trois ampoules de cellules VERO, les résultats obtenus sont résumés ci-dessous.

**Tableau 1**

| | Jour 2 | Jour 5 |
|---|---|---|
| | embryon 2 cellules - /- zygote | Blastocyste -/- embryon 2 cellules |
| Ampoule 1 | 97% | 96% |
| Ampoule 2 | 97% | 96% |
| Ampoule 3 | 97% | 97% |
| *moyenne* | 97% | 96% |

Les résultats obtenus montrent que le système de décongélation des cellules VERO par les filtres à centrifuger permet de produire un tapis cellulaire apte au développement des embryons de souris jusqu'au stade blastocyste, et ces derniers sont également aptes à éclore.

Le taux de blastulation sur le tapis cellulaire de cellules Véro est très satisfaisant : plus de 90%.

### Exemple 3 : congélation/décongélation de cellules dans un même et unique contenant : tube à centrifuger ultrafree

Des cellules Véro fraîchement trypsinées sont congelées directement dans la colonne à filtre du tube Ultrafree (souvent appelée "le filtre" par abus de langage). Les cellules sont ensuite décongelées dans ces mêmes tubes.

### Matériels :

- Description des filtres à centrifuger : *cƒ* exemple 1
- Processus préalable sur les filtres à centrifuger : *cƒ* exemple 1
- le glycérol utilisé est celui de Euromedex, ref 50405, pureté > 99.5%, grade biologie moléculaire et élèctrophorèse. Du glycérol de culture cellulaire peut bien sûr être utilisé à la place de celui utilisé ici, de même que n'importe quel liquide non miscible avec l'eau et compatible avec la survie des cellules / la culture cellulaire.
- Cellules : lignée cellulaire de rein de singe : cellules Véro, cryoconservées en vapeur d'azote, à 2×10⁶ cell/ampoule, avec 1ml de milieu de congélation : milieu de culture MEMα (80%) / Sérum de Veau Fœtal (10%) / Dimethyl sulfoxide (10%)
- Mise en culture des cellules : milieu de culture MEMa (95%) / Sérum de Veau Fœtal (5%)

### Protocole :

*Congélation de la suspension cellulaire dans le tube Ultrafree contenant le filtre à centrifuger* A partir d'une suspension de cellules en culture fraîchement trypsinées (viabilité mesurée = viabilité avant congélation) :
- préparer une suspension à 0.5 millions /ml dans du milieu de congélation composé de 70% MEM-α (Gibco), 20% SVF, 10 % DMSO ;
- Déposer 50µl de glycérol sur le filtre à centrifuger ;
- Déposer par-dessus 1ml de la suspension cellulaire dans le milieu de congélation ;
- Réalisation de 3 tubes à centrifuger de cette façon ;
- Fermer les tubes Ultrafree et les déposer à -80°C.

*Décongélation des cellules cryoconservées dans le même tube Ultrafree contenant le filtre à centrifuger*
- Placer les 3 tubes Ultrafree contenant les suspensions cellulaires, 1 minute au bain marie à 37°C ;
- Centrifuger les tubes 5 min à 100 g ;

*Récupération des cellules sur le filtre des tubes Ultrafree à la fin de la centrifugation :*
- Déposer 500µL de milieu de culture 95% MEM-α (Gibco), 5% SVF sur le filtre tube Ultrafree ;
- Réaliser une série de 5 cycles aspiration/refoulement sur la totalité du filtre :
- Déposer à nouveau 500µL de milieu de culture sur le filtre tube Ultrafree ;
- Réaliser une série de 5 cycles aspiration/refoulement sur la totalité du filtre ;
- 1ml final de la suspension cellulaire est mis dans un tube de 15ml
- Prélever et déposer 100 µL de la suspension cellulaire pour comptage
- Réaliser le comptage cellulaire, et viabilité, en déposant de la suspension cellulaire entre lame et lamelle d'un hématimètre
- Ensemencer en flask de 75cm² et incuber les plaques à 37°C+5%CO₂

### Résultat :

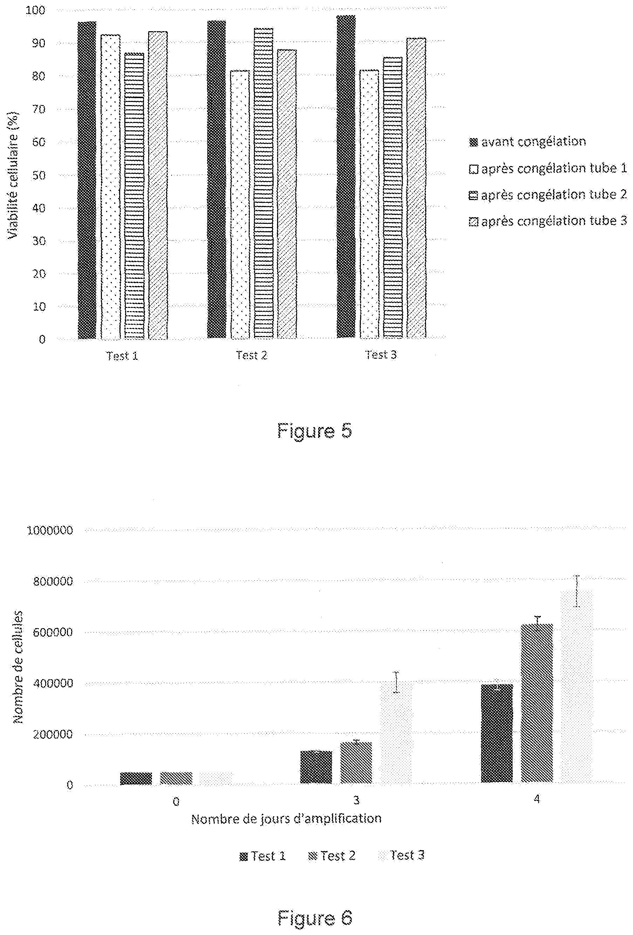

L'ensemble de l'essai est reproduit trois fois, les résultats de viabilité sont reportés dans la figure 5. Une viabilité de l'ordre de 88.0 % (± 4.9) a été obtenue. La figure 6 confirme que cette technique est fiable et reproductible, les cellules montrant une bonne capacité de prolifération après décongélation.

## Revendications

1. Procédé de décongélation cellulaire, comprenant une étape de décongélation d'une suspension cellulaire constituée d'un milieu de congélation et de cellules, suivie d'une étape d'élimination dudit milieu de congélation par filtration, ladite étape d'élimination du milieu de congélation étant effectuée sans apport préalable ou simultané de milieu, ledit procédé comprenant les étapes suivantes :
(i) placer un récipient, contenant une suspension cellulaire congelée dans un dispositif muni d'un filtre, à une température permettant la décongélation de la suspension cellulaire ;
(ii) appliquer à la suspension cellulaire une force qui entraîne le passage du milieu de congélation à travers le filtre ;
(iii) remettre les cellules en suspension dans une solution.

2. Procédé selon la revendication 1, **caractérisé en ce que** le dispositif muni d'un filtre est un filtre à centrifuger.

3. Procédé selon la revendication 1 ou la revendication 2, **caractérisé en ce que** le diamètre moyen des pores du filtre est compris entre 2 et 10 microns.

4. Procédé selon l'une quelconque des revendications précédentes, **caractérisé en ce que** le passage du milieu de congélation à travers le filtre est obtenu en centrifugeant le dispositif muni d'un filtre, placé dans le récipient.

5. Procédé selon la revendication 4, **caractérisé en ce que** le dispositif est centrifugé pendant une durée inférieure ou égale à 10 minutes, à une vitesse permettant d'appliquer à la suspension cellulaire une accélération comprise entre 50 et 1500 g, de préférence entre 100 et 200 g.

6. Procédé selon l'une quelconque des revendications 1 à 3, caractérisé en que le passage du milieu de congélation à travers le filtre est obtenu en appliquant une surpression du côté du filtre contenant la suspension cellulaire, et/ou une dépression de l'autre côté du filtre.

7. Procédé selon l'une quelconque des revendications 1 à 6, **caractérisé en ce que** la quantité de cellules placées dans chaque dispositif muni d'un filtre est comprise entre 10⁴ et 10⁷.

8. Procédé selon l'une quelconque des revendications précédentes, **caractérisé en ce que** l'étape de remise en suspension des cellules est réalisée en effectuant des pipetages à l'aide d'une pipette P200 ou P1000.

9. Procédé selon l'une quelconque des revendications 1 à 8, dans lequel le filtre est hydrophobe, ou a été recouvert, avant l'ajout de la suspension cellulaire à congeler, d'un liquide hydrophobe constituant une barrière transitoire entre le filtre et la suspension cellulaire, ledit liquide étant éliminé à l'étape (ii) lors de l'application de la force d'entraînement.

10. Trousse de décongélation cellulaire, comprenant un récipient contenant une suspension cellulaire congelée dans un dispositif muni d'un filtre dont les pores ont un diamètre moyen compris entre 1 et 15 microns, ledit dispositif étant stérile.

11. Trousse selon la revendication 10, **caractérisée en ce que** les cellules dans la suspension cellulaire sont des cellules VERO.

12. Trousse selon l'une quelconque des revendications 10 à 11, **caractérisée en ce que** le dispositif muni d'un filtre est un filtre à centrifuger.

13. Trousse selon l'une quelconque des revendications 10 à 12, **caractérisée en ce que** le diamètre moyen des pores du filtre est compris entre 2 et 10 microns.

14. Trousse selon l'une quelconque des revendications 10 à 13, destinée à favoriser la culture d'embryons ou la maturation *in vitro* d'ovocytes, **caractérisée en ce qu'**elle comprend en outre un ou plusieurs des éléments suivants : des pointes stériles de 200µl et/ou 1000µl, un support de culture, des tubes stériles, des pipettes stériles.

15. Trousse selon l'une quelconque des revendications 10 à 14, destinée à favoriser la culture d'embryons ou la maturation *in vitro* d'ovocytes, **caractérisée en ce qu'**elle comprend en outre un milieu de rinçage et/ou un milieu de culture approprié.

## Patentansprüche

1. Verfahren zum Auftauen von Zellen, umfassend einen Schritt des Auftauens einer Zellsuspension, die aus einem Gefriermedium und Zellen besteht, gefolgt von einem Schritt des Entfernens des Gefriermediums durch Filterung, wobei der Schritt des Entfernens des Gefriermediums ohne vorherige oder gleichzeitige Zufuhr von Medium durchgeführt wird,
wobei das Verfahren die folgenden Schritte umfasst:
(i) Bereitstellen eines Behälters, der eine gefrorene Zellsuspension in einer mit einem Filter versehenen Vorrichtung enthält, bei einer Temperatur, die das Auftauen der Zellsuspension ermöglicht;
(ii) Aufbringen einer Wirkkraft auf die Zellsuspension, die bewirkt, dass das Gefriermedium durch den Filter hindurchtritt;
(iii) Resuspendieren der Zellen in einer Lösung.

2. Verfahren nach Anspruch 1,
**dadurch gekennzeichnet, dass** die mit einem Filter versehene Vorrichtung ein Zentrifugenfilter ist.

3. Verfahren nach Anspruch 1 oder Anspruch 2,
**dadurch gekennzeichnet, dass** der mittlere Durchmesser der Poren des Filters zwischen 2 und 10 Mikron liegt.

4. Verfahren nach einem der vorhergehenden Ansprüche,
**dadurch gekennzeichnet, dass** der Durchtritt des Gefriermediums durch den Filter durch Zentrifugieren der mit einem Filter versehenen Vorrichtung, die in den Behälter eingesetzt ist, erreicht wird.

5. Verfahren nach Anspruch 4,
**dadurch gekennzeichnet, dass** die Vorrichtung für eine Zeitdauer kürzer oder gleich 10 Minuten mit einer solchen Geschwindigkeit zentrifugiert wird, dass auf die Zellsuspension eine Beschleunigung zwischen 50 und 1500 g, vorzugsweise zwischen 100 und 200 g, ausgeübt wird.

6. Verfahren nach einem der Ansprüche 1 bis 3,
**dadurch gekennzeichnet, dass** der Durchtritt des Gefriermediums durch den Filter durch Aufbringen eines Überdrucks auf der Seite des Filters, die die Zellsuspension enthält, und/oder eines Unterdrucks auf der anderen Seite des Filters erreicht wird.

7. Verfahren nach einem der Ansprüche 1 bis 6,
**dadurch gekennzeichnet, dass** die Menge der Zellen, die in jede mit einem Filter versehene Vorrichtung eingesetzt wird, zwischen 10⁴ und 10⁷ beträgt.

8. Verfahren nach einem der vorhergehenden Ansprüche,
**dadurch gekennzeichnet, dass** der Schritt des Resuspendierens der Zellen durch Pipettieren mit einer Pipette P200 oder P1000 erfolgt.

9. Verfahren nach einem der Ansprüche 1 bis 8,
wobei der Filter hydrophob ist oder vor der Zugabe der zu gefrierenden Zellsuspension mit einer hydrophoben Flüssigkeit beschichtet wurde, die eine vorübergehende Barriere zwischen Filter und Zellsuspension bildet, wobei diese Flüssigkeit in Schritt (ii) beim Aufbringen der Wirkkraft entfernt wird.

10. Kit zum Auftauen von Zellen, bestehend aus mindestens einem Behälter, der eine gefrorene Zellsuspension in einer Vorrichtung enthält, die mit einem Filter versehen ist, dessen Poren einen mittleren Durchmesser zwischen 1 und 15 Mikron aufweisen, wobei die Vorrichtung steril ist.

11. Kit nach Anspruch 10,
**dadurch gekennzeichnet, dass** die Zellen in der Zellsuspension Vero-Zellen sind.

12. Kit nach einem der Ansprüche 10 bis 11,
**dadurch gekennzeichnet, dass** die mit einem Filter versehene Vorrichtung ein Zentrifugenfilter ist.

13. Kit nach einem der Ansprüche 10 bis 12,
**dadurch gekennzeichnet, dass** der mittlere Durchmesser der Poren des Filters zwischen 2 und 10 Mikron liegt.

14. Kit nach einem der Ansprüche 10 bis 13, das dazu bestimmt ist, die Kultur von Embryonen oder die In-vitro-Reifung von Eizellen zu fördern, **dadurch gekennzeichnet, dass** es ferner eines oder mehrere der folgenden Elemente umfasst: sterile Spitzen von 200 µl und/oder 1000 µl, einen Kulturträger, sterile Röhrchen, sterile Pipetten.

15. Kit nach einem der Ansprüche 10 bis 14, das dazu bestimmt ist, die Kultur von Embryonen oder die In-vitro-Reifung von Eizellen zu fördern, **dadurch gekennzeichnet, dass** es ferner ein Spülmedium und/oder ein geeignetes Kulturmedium umfasst.

## Claims

1. A cell thawing method comprising a step of thawing a cell suspension consisting of a freezing medium and of cells, followed by a step of removing said freezing medium by filtration, said step of removing the freezing medium being carried out without prior or simultaneous provision of medium, said method comprising the following steps:
(i) placing a container, containing a frozen cell suspension in a device equipped with a filter, at a temperature which allows the cell suspension to thaw;
(ii) applying to the cell suspension a force which drives the passing of the freezing medium through the filter;
(iii) resuspending the cells in a solution.

2. The method as claimed in claim 1, **characterized in that** the device equipped with a filter is a centrifugal filter.

3. The method as claimed in claim 1 or claim 2, **characterized in that** the average diameter of the pores of the filter is between 2 and 10 microns.

4. The method as claimed in any one of the preceding claims, **characterized in that** the passing of the freezing medium through the filter is obtained by centrifuging the device equipped with a filter, placed in the container.

5. The method as claimed in claim 4, **characterized in that** the device is centrifuged for a period of less than or equal to 10 minutes, at a speed which makes it possible to apply to the cell suspension an acceleration of between 50 and 1500 g, preferably between 100 and 200 g.

6. The method as claimed in any one of claims 1 to 3, **characterized in that** the passing of the freezing medium through the filter is obtained by applying an increased pressure on the side of the filter containing the cell suspension, and/or a reduced pressure on the other side of the filter.

7. The method as claimed in any one of claims 1 to 6, **characterized in that** the amount of cells placed in each device equipped with a filter is between 10⁴ and 10⁷.

8. The method as claimed in any one of the preceding claims, **characterized in that** the step of resuspending the cells is carried out by performing pipetting operations using a P200 or P1000 pipette.

9. The method as claimed in any one of claims 1 to 8, wherein the filter is hydrophobic, or, before the addition of the cell suspension to be frozen, has been covered with a hydrophobic liquid forming a temporary barrier between the filter and the cell suspension, said liquid being removed in step (ii) during the application of the driving force.

10. A cell thawing kit comprising a container containing a frozen cell suspension in a device equipped with a filter, the pores of which have an average diameter of between 1 and 15 microns, said device being sterile.

11. The kit as claimed in claim 10, **characterized in that** the cells in the cell suspension are VERO cells.

12. The kit as claimed in any one of claims 10 to 11, **characterized in that** the device equipped with a filter is a centrifugal filter.

13. The kit as claimed in any one of claims 10 to 12, **characterized in that** the average diameter of the pores of the filter is between 2 and 10 microns.

14. The kit as claimed in any one of claims 10 to 13, intended for promoting the embryo culture or oocyte *in vitro* maturation, **characterized in that** it also comprises one or more of the following elements: 200 µl and/or 1000 µl sterile tips, a culture support, sterile tubes and sterile pipettes.

15. The kit as claimed in any one of claims 10 to 14, intended for promoting the embryo culture or oocyte *in vitro* maturation, **characterized in that** it also comprises a rinsing medium and/or an appropriate culture medium.
